# EUROPEAN PATENT SPECIFICATION

(11) **EP 2 200 690 B1**
(45) Date of publication and mention of the grant of the patent: **14.11.2012**
(21) Application number: 08807702.9
(22) Date of filing: 17.09.2008
(51) Int. Cl.: A61M 35/00, A61L 26/00

(54) **SYSTEM FOR APPLYING A SKIN SEALANT HAVING A PHASE CHANGE VISUAL INDICATING COMPONENT**
SYSTEM ZUM AUFBRINGEN EINES HAUTDICHTUNGSMITTELS MIT VISUELLER PHASEN-VERÄNDERUNGS-ANZEIGEKOMPONENTE
SYSTÈME POUR APPLIQUER UN FILM DE PROTECTION CUTANÉE AYANT UN COMPOSANT D'INDICATION VISUELLE DE CHANGEMENT DE PHASE

(30) Priority: 12.10.2007 US 974393
(43) Date of publication of application: 30.06.2010
(73) Proprietor: KIMBERLY-CLARK WORLDWIDE, INC., Neenah, WI 54956 (US)
(72) Inventor: MACDONALD, John, Gavin, Decatur Georgia 30033 (US); SMITH, Molly, K., Atlanta Georgia 30317 (US); WEART, Ilona, F., Woodstock Georgia 30188 (US); SCHORR, Phillip, A., Atlanta Georgia 30350 (US)
(74) Representative: Davies, Christopher Robert
(86) International application number: PCT/IB2008/053779
(87) International publication number: WO 2009/047663

(56) References cited:
- WO-A2-02/30363
- US-A- 4 621 029
- US-A- 4 996 292
- US-A- 5 403 336
- US-A1- 2003 065 069
- US-A1- 2007 147 947
- US-B1- 6 238 373

## Description

Surgical site infections (SSI) occur following about 2-3 percent of surgeries in the United States with an estimated 500,000 incidents of SSI occurring annually, which can lead to significant patient morbidity and mortality. In addition to the negative impact of such infections on patient health, these potentially avoidable infections contribute significantly to the financial burden experienced by the health care system. SSIs result when an incision becomes contaminated by bacteria, and for most surgeries the primary source of these infection-causing microorganisms is the skin (an exception being surgeries in which the gastrointestinal tract is penetrated).

Various compositions are used to prepare the skin prior to surgery. Skin preparations or "preps" are used to remove some level of microbial load on the skin prior to making an incision. Skin sealant materials are used to protect patients from bacterial infections associated with surgical site incisions and insertion of intravenous needles. Skin preps are applied to the skin and allowed to dry to maximize effectiveness for reducing microorganisms. After the skin prep has dried, the sealant may be applied directly to the skin in liquid form. The sealant forms a coherent film with strong adhesion to the skin through various techniques based on the chemistry of the sealant.

Skin preps currently are predominantly povidone-iodine or chlorhexidine gluconate based formulations and may contain alcohol for fast drying and more effective killing of organisms. Time constraints in the operating room and the lack of an indicator that the prep has dried often result in the skin remaining wet when draping and/or surgery begin, creating the possibility of infection. The lack of an indicator can also negatively impact infection since the users cannot know with certainty where the prep and sealant have been applied.

Skin sealants now use a polymer composition that dries to form a film through evaporation of a solvent, for example. Other skin sealants contain monomeric units that polymerize in situ to from a polymeric film. Cyanoacrylate sealants containing alkyl cyanoacrylate monomer are an example of the latter type wherein the monomer polymerizes in the presence of a polar species such as hydroxide, water or protein molecules to form an acrylic film. The resulting film serves to immobilize bacterial flora found on the skin and prevents their migration into an incision made during a surgical procedure or skin puncture associated with insertion of an intravenous needle.

In addition to the film former, skin sealants contain plasticizing agents to improve film flexibility and conformance. This is desirable in skin sealant applications in order to prevent the film from cracking or flaking off during use and so that the film is flexible enough to allow for movement or adjustment of the limb or appendage without compromising the barrier properties of the sealant film. They may also include viscosity modifiers to aid in application of the liquid composition, free radical and anionic scavengers to stabilize the product prior to use, biocidal agents to kill immobilized bacteria under the film, and the like.

Skin sealants have also been formulated with colorants to help the user apply the liquid composition uniformly to the skin, especially when large areas are to be covered. There are several problems, however, with existing colorants; addition of a colorant directly to the liquid skin sealant composition can negatively impact both in situ polymerization rates and the conversion reaction, in the case of cyanoacrylate compositions, or evaporation rates and the coalescence process in the case of polymer solution compositions. In addition, known colorants do not provide a visual cue to indicate curing of the composition has been completed. Lastly, after completion of the surgical procedure, the colorant in the sealant can obscure the wound site, making it difficult to detect redness associated with surgical site infections, bruising or leakage.

It is clear that there exists a need for a colorant that provides a visual cue to indicate coverage area and/or curing and that does not obscure the wound site. U.S. 2007/0147947 discloses a system for sealing skin according to the preamble of claim 1.

### SUMMARY OF THE INVENTION

In accordance with the present invention there is provided a system for sealing skin with a film forming polymer as set forth in claim 1.

In response to the foregoing difficulties encountered by those of skill in the art, we have discovered that plasticized film formers including various color changing dyes may be used to indicate that the skin sealant composition has dried. The dyes change color in response to the phase change, i.e., drying of the skin sealant. The dye may be added either directly to the film former (which may contain an added plasticizer), incorporated into a sponge on the applicator through which plasticized film former is dispensed and applied, applied separately or applied simultaneously from a separate reservoir. The amount of dye in the film former composition can be adjusted to provide a visual cue to the user of the application area and the extent of cure and, in some formulations, to act as a plasticizer. Generally speaking, dyes include xanthene dyes such as, for example, Drug & Cosmetic (D&C) orange 5 and D&C Orange 10.

### DESCRIPTION OF THE DRAWINGS

Figure 1 is a drawing of a skin sealant applicator that may be used in the practice of the system.
Figure 2 includes two views. Figure 2A is a drawing of an applicator in a first position and Figure 2B is a drawing of an applicator in a second position.
Figure 3 is a drawing of an applicator having an instructive arrow on an outside surface.

### DETAILED DESCRIPTION OF THE INVENTION

Skin preparations or "preps" are used to remove some level of microbial load on the skin prior to making an incision. Skin preps are applied to the skin and allowed to dry to maximize effectiveness for reducing microorganisms. Skin preps currently are predominantly povidone-iodine or chlorhexidine gluconate based formulations and may contain alcohol for fast drying and more effective killing of organisms. Povidone iodine, available commercially as Betadine® is estimated to be used in 80 percent of surgeries as a skin preparation. Betadine® skin prep is an aqueous solution of 10 percent povidone iodine having 1 percent titratable iodine content. When Betadine® skin prep is applied to the skin, it imparts and orange-brown color. Chloraprep® skin prep is another common formulation and is chlorhexidine gluconate based.

Skin sealant materials are used to protect patients from bacterial infections associated with surgical site incisions and insertion of intravenous needles. Skin sealants are often applied directly over or on top of skin preps. The sealant forms a coherent film with strong adhesion to the skin through various techniques based on the chemistry of the sealant composition. The skin sealants used herein contain a film former and a plasticizer and other optional ingredients like viscosity modifiers to aid in application of the liquid composition, free radical and anionic scavengers to stabilize the product prior to use, biocidal agents to kill immobilized bacteria under the film, and the like.

One film former available in a skin sealant composition is commercially known as InteguSeal® and is available from Medlogic Global, Ltd of Plymouth, England. InteguSeal® skin sealant contains medical grade n-butyl cyanoacrylate monomer (80 % w/w). Medical grade cyanoacrylate is double distilled. Non-medical grade cyanoacrylate, in contrast, is single distilled and is typically marketed as a "super glue" type adhesive for gluing a wide variety of substrates together. Another film former is Hard as Nails@ tosylamide/formaldehyde based-resin (Del Laboratories Inc., Uniondale NY).

The use of plasticizer in the skin sealant is important in order to give the film enough flexibility and conformability in order prevent cracking or flaking of the coating. The range of plasticizer required is about 10 to 60% wt/wt, more particularly between 15 and 50% and most particularly between 18 and 25% wt/wt. Common plasticizers that may be used include tributyl o-acetyl citrate, acetin, other alkyl substituted citrate derivatives, dioctylphthalate and acrylic acid monomer. It should be noted that at higher concentrations (>3000ppm) the dye itself can act as a plasticizer giving improved properties to the cured film. The inventors surmise that the plasticizer effect of the dye is probably due to the surface active agent properties of the dye. Parts per million ("ppm") denotes one particle of a given substance for every 999,999 other particles. This is roughly equivalent to one drop of ink in a 150 liter (40 gallon) drum of water, or one second per 280 hours (11 days, 16 hours). One part in 10⁶ — a precision of 0.0001 %.

Generally speaking, the cyanoacrytate can be mixed with the dye using a stir bar or mechanical stirrer while warmed to and held at 60 °C until the dye dissolved (5-20 minutes). The heating is important with dye concentrations of 500 ppm and greater in order to dissolve the dye. Without heating, the majority of the dye would settle out as a precipitate. Once heated, the dye will remain in solution even after cooling to ambient temperature or storing at refrigerator or freezer temperatures. The composition should be heated at a temperature and for a time sufficient to dissolve the dye. For many of the dyes contemplated by the present invention, the heating should be, for example, between 50 and 70 °C for at least 5 minutes. Alternatively the dye may be dissolved in a plasticizer and the resultant solution blended with cyanoacrylate in the appropriate proportion to produce the desired dye concentration in the final composition. A somewhat higher temperature is believed to be necessary to dissolve the dye in the plasticizer. As yet another alternative, the dye may be mixed with a fugitive solvent and the resultant solution combined with the cyanoacrylate and the solvent allowed to escape (e.g. by evaporation) to produce the final composition..

The intensity or brightness of light is expressed in lux (Ix), for example, an over cast summer day is estimated to between 30,000Ix and 40,000Ix and a mid-winter day is estimated to be about 1 0,000Ix. The British Standards Institution Code of Practice for Day-lighting, BS 8206 Part 1 deals in general terms with the code of practice for artificial light. The following gives some general guidance for the light requirements for the work place.
- General office, laboratories, kitchen - 500Ix
- Drawing offices - 7501x
- Tool rooms and paintwork - 1000Ix
- Inspection of graphic reproduction - 1500Ix.
Accordingly, for purposes of the present invention "normal light conditions" refers to light conditions of between about 500Ix and 2000Ix, more desirably, from about 750Ix to about 1500Ix as determined in accordance with BS 8206 part 1.

It would be useful to medical personnel to know exactly where the skin sealant and prep were applied so that they could be sure that the appropriate area was covered. The inventors believe that providing a skin sealant and/or skin prep which will change color as it dries will provide valuable information for the medical professional.

A unique set of dyes were identified that provide a novel indication of both coverage and cure visible by the unaided human eye or otherwise optically discernable by scanners or other optical equipment that operate under normal lighting conditions, i.e., general office, laboratories and kitchens and not requiring ultraviolet light (UV wavelengths below 400 nanometers to about 200 nanometers) or "black light" or other specialized lighting. The improved coverage indicator allows the user to clearly see where they have applied the coating via a highly visible color, and additionally can indicate proper coating thickness via the intensity of the color. If the coating is applied too thinly there will be a near lack of color, and where it is applied too thickly it will be more intensely colored. It is also desired that the color of this coverage indicator be such that skin tone and surgical markings on the skin are visible through it and not mistaken for a physiological condition such as skin bruising, rash, or infection. In this manner the coating and dye allow the surgeon to see their markings at the surgical site before surgery and allows for evaluation of wound health after surgery.

The inventors have identified a unique class of dyes and demonstrated in skin sealant and other curable resins a vivid color change, signaling both the coverage and the completion of the resin's cure. They have found these dyes to be sensitive to the phase change that occurs when an adhesive cures. The dyes identified all belong to, and are a subset of, the xanthene class of dyes, whose structure is given below. 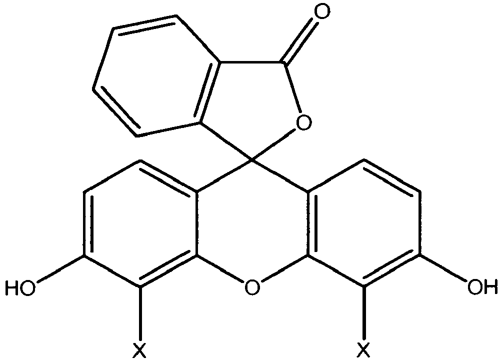 Where X is a halogen

They are fluorescent by nature of their chromophore and rely upon microcrystal interactions for their intense colors. While not wishing to be held to a particular theory, it is believed that as the sealant cures the polymer chains disrupt this microcrystal interaction, thereby changing the observed color. The dye range was 3000 to 10,000ppm, particularly 3500 to 8,000ppm and most particularly 4000 to 6000ppm.

Other cure indicators making use of pH indicators have been described in the art. There is a change in pH during cure of cyanoacrylate systems typically from pH 2 to pH 3 or 4. Unfortunately none of these indicators have been approved by the US Food and Drug Administration for direct skin contact. The phase sensing dyes are not pH indicators and do not change color within the pH range observed during the skin sealant's cure.

The term "phase change" used herein has its conventional meaning in the art, that is; it is a change in physical state of a substance, in this case, from a liquid to a solid. The term "phase transformation" may be used interchangeably to refer to a change of a substance from one phase to another. Here the inventors define a solid as a flexible solid or a gel or a non-flexible solid.

The phase change dyes are believed to take advantage of aggregation, and it was demonstrated to occur in cyanoacrylate and other curing resin systems by the inventors. The dyes are sensitive enough to detect and indicate the phase change that occurs as the sealant composition cures. It is known to those skilled in the art that fluorescent molecules behave differently in liquid solutions than they do in solid states. It is believed that the reason has to do with whether the dye molecules are present as freely floating single molecule units or as excimers (aggregates of several molecules stacked together). Solutions are predominantly monomers and solids contain mostly aggregates. Typically, aggregation causes quenching of dye fluorescence and a shifting of the emission spectrum so solid systems show weaker fluorescence of a different color than do liquid systems of the same dye. However, in this invention as the resin monomer polymerizes the dyes form excimers which cause the change in color and/or the increase in fluorescence.

As noted above, there a number of ways to use a dye with a film former: it may be mixed with the skin sealant in sufficient quantities to plasticize the film former, it may be mixed with a composition of plasticizer and film former, it may be impregnated onto a sponge or wipe which is used to apply the sealant, it may be applied separately from a separate reservoir and it may be applied simultaneously from a separate reservoir in a manner similar to the application of an epoxy.

The application of a dye to a carrier may be done by the "dip and squeeze" method, known to those skilled in the art. In this method, the carrier (e.g., sponge, nonwoven fabric (wipe), cotton ball or other) is placed in a bath of the dye and allowed to absorb the dye. After absorbing the dye, the carrier is squeezed between, for example, a pair of rollers, to force out excess dye.

Another method to apply dye to a carrier is to spray the dye onto the carrier. Spraying generally does not penetrate the carrier with dye as well as the dip and squeeze method, though it is generally faster and simpler.

Yet another method to apply a dye to, for example, a stack of wipes in a storage box, is to add the dye to the box with the wipes. US patents 4,775,582 and 4,853,281, concern a method of maintaining relatively uniform moisture in a stack of wipes. The wipes may be made from polyolefinic microfibers that have been extruded and gathered like spunbond or meltblown fibers, or a combination of both. Common materials for construction of wipers include spunbond and meltblown fibers and fabrics in various arrangements.

The term "spunbond fibers" refers to small diameter fibers which are formed by extruding molten thermoplastic material as filaments from a plurality of fine, usually circular capillaries of a spinneret with the diameter of the extruded filaments then being rapidly reduced as by, for example, in US Patent 4,340,563 to Appel et al., and US Patent 3,692,618 to Dorschner et al., US Patent 3,802,817 to Matsuki et al., US Patents 3,338,992 and 3,341,394 to Kinney, US Patent 3,502,763 to Hartman, and US Patent 3,542,615 to Dobo et al. Spunbond fibers are generally not tacky when they are deposited onto a collecting surface. Spunbond fibers are generally continuous and have average diameters (from a sample of at least 10) larger than 7 microns, more particularly, between about 10 and 20 microns. As used herein the term "meltblown fibers" means fibers formed by extruding a molten thermoplastic material through a plurality of fine, usually circular, die capillaries as molten threads or filaments into converging high velocity, usually hot, gas (e.g. air) streams which attenuate the filaments of molten thermoplastic material to reduce their diameter, which may be to microfiber diameter. Thereafter, the meltblown fibers are carried by the high velocity gas stream and are deposited on a collecting surface to form a web of randomly dispersed meltblown fibers. Such a process is disclosed, for example, in US Patent 3,849,241 to Butin et al. Meltblown fibers are microfibers which may be continuous or discontinuous, are generally smaller than 10 microns in average diameter, and are generally tacky when deposited onto a collecting surface. Laminates of spunbond and meltblown fibers may be made, for example, by sequentially depositing onto a moving forming belt first a spunbond fabric layer, then a meltblown fabric layer and last another spunbond layer and then bonding the laminate in a manner described below. Alternatively, the fabric layers may be made individually, collected in rolls, and combined in a separate bonding step. Such fabrics usually have a basis weight of from about 0.1 to 12 osy (6 to 400 gsm), or more particularly from about 0.75 to about 3 osy. Multilayer laminates may also have various numbers of meltblown (abbreviated as "M") layers or multiple spunbond (abbreviated as "S") layers in many different configurations and may include other materials like films (abbreviated as "F") or coform materials (see US 4,100,324 for descriptions of exemplary "coform" materials), e.g. SMMS, SM, SFS, etc.

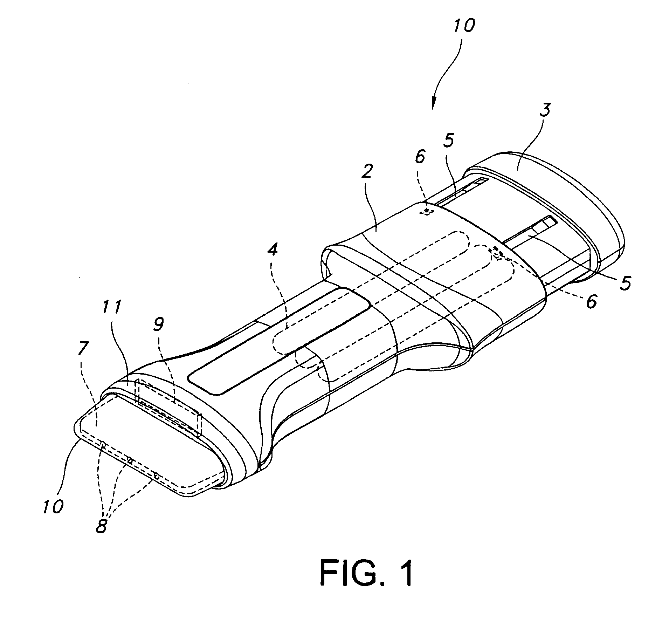

Applying the sealant from a separate reservoir may involve the use of dispensers developed for that purpose. One exemplary dispenser 10 as shown in Figure 1 has the liquid sealant composition held in at least one oblong glass ampoule 4 within a rigid nylon housing. The housing has a body 2 and a cap 3 that are slidably connected and it is the cap 3 which holds the ampoule(s) 4. In use, the two parts are moved toward each other to dispense the liquid; the cap 3 moving from a first position into a second position within the body 2. Moving the parts together results in breakage of the frangible glass ampoule(s) 4 and dispensing of the liquid. A detent-type locking mechanism holds the body and cap together once they are moved. The locking mechanism consists of slots 5 formed in the cap 3 into which fits a slight protuberance or knoll of plastic 6 formed on the inside surface of the body 2. Once the ampoule is broken, the liquid travels through a small piece of foam 9 which catches any glass shards that may have been formed by the breakage of the ampoule and thence on to the tip 7 portion of the body. The tip 7 has a number of small holes 8 in it to allow the liquid to pass through. The body tip has a piece of foam 10 on the outside, held in place with a rigid plastic oval-shaped ring 11 that snaps in place on the tip 7. The outer foam 10 contacts the skin of the patient when the liquid is dispensed. See for example US patent publication 20070147947. Other types of dispensers may be found in US patents 4,854,760, 4,925,327 and 5,288,159.

The system for sealing skin with a film forming polymer disclosed herein involves providing an at least two part applicator containing the sealant composition in one or more frangible containers, providing instructions for using the applicator associated therewith, where the instructions include moving at least one of the applicator parts from a first position to a second position and applying the sealant to skin. The composition has a film former, a plasticizer and 3000 to 10000 ppm of a dye that changes color when the composition undergoes a phase change, and the color change is visible to an unaided human eye under normal light conditions.

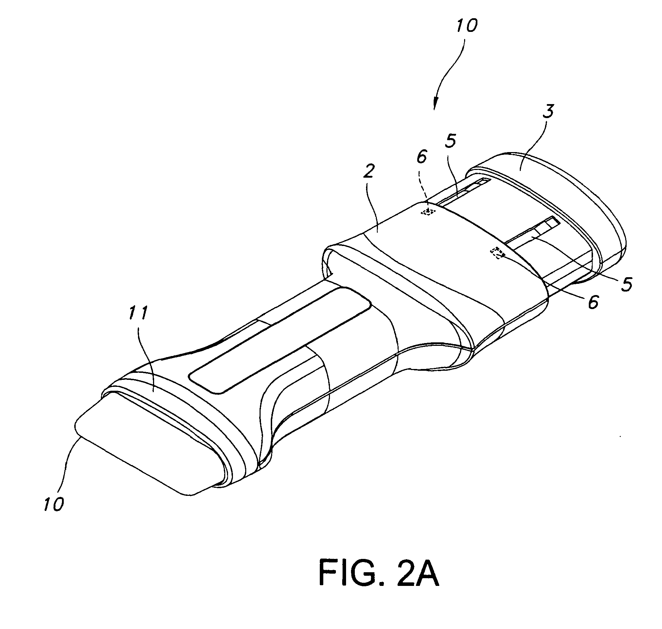

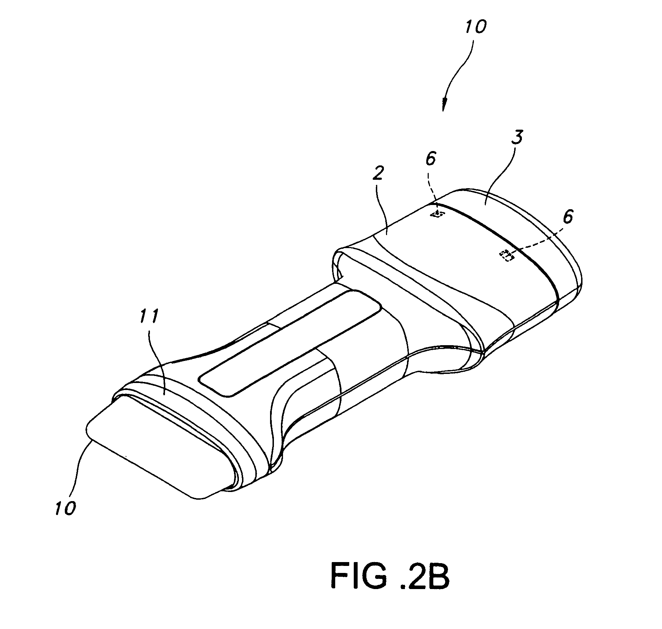

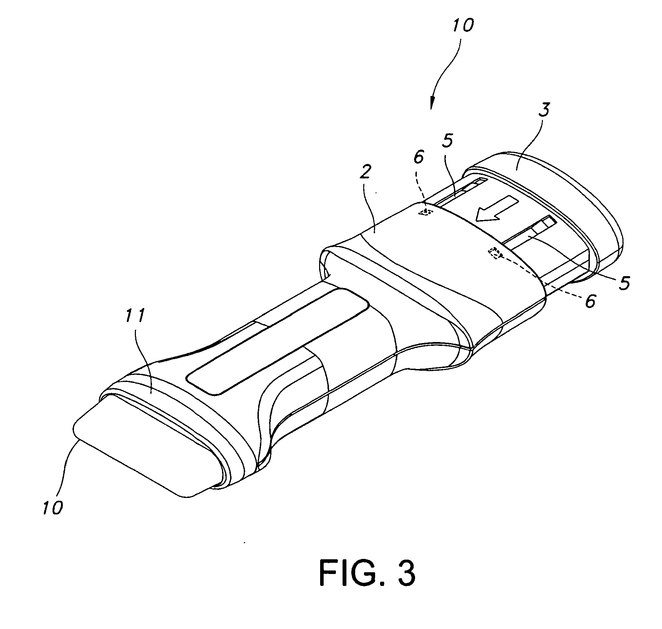

The instructions provided are for example, an illustration of the applicator with the parts in the first and second positions (see Figures 2A and 2B) or an instructive arrow on the outer surface of the applicator (see Figure 3) indicating in which direction to move the parts from the first to second postions.

The applicator may be packaged in such a way as to provide an article of manufacture where the packaging features a brand name and/or logo of a skin prep composition, e.g. Betadine®, Chloraprep®, that the sealant may be used with. This provides instruction to the medical professional and helps ensure that the sealant composition is used properly.

In another example the plasticized skin sealant and dye may be applied separately to the area containing a skin prep. US patent 5,928,611 describes a dispenser having a sealant reservoir and an active ingredient such as a cross linking accelerator or initiator disposed on a foam piece through which the sealant must pass. One could envision the use of such a dispenser having the dye disposed on the foam piece and the sealant passing though it as it is about to be deposited onto the skin. See also US patent 6,322,852.

In yet another example, US patent 6,340,097 describes a dispenser having at least one crushable ampoule within the body of the dispenser (which could hold more than one). This would permit one ampoule to hold plasticized skin sealant and a second to hold the dye. When the dispenser was used, it would break both ampoules and the sealant composition and dye would mix just before application to the skin.

In addition to being used as a traditional skin sealant, i.e. as a plasticized film forming barrier through which a surgical incision is made, the dye and skin sealant composition may also be used like a bandage to close and/or cover wounds, abrasions, bums, acne, blisters and other disruptions in the skin to protect them from subsequent contamination. The use of the skin sealant composition would therefore not be limited to medical personnel.

Wound protection is critical in permitting the healing process to take place. Traditional adhesive bandages and gauze wound dressings have been used by the consumer to treat/dress acute wounds or skin irritations. Such adhesive bandages are generally passive, in that they offer little or no chemical treatment for wound healing. Rather, they primarily serve to exert low levels of pressure on the wound, protect the wound from exposure to the environment, and absorb any exudates, which are produced from the wound site. Such bandages generally include a base layer, which is the layer seen by the consumer following application of the bandage to the wound. Such a layer is typically formed from a polymeric material such as a film, nonwoven web, or combination thereof, and may be perforated in some fashion to allow for flexibility and/or further breathability. This layer often includes a film component, having a top side surface which is seen by the consumer after application of the bandage to the wound site, and a bottom side surface (skin contacting surface). A skin-friendly adhesive is usually placed over the base layer bottom side surface to provide a means for attaching the bandage to the consumer. Alternatively, a separate adhesive tape is used to attach the bandage/wound dressing to the wound site, if the bandage/wound dressing is of the nonadhesive type. In the center of the base layer bottom side surface is traditionally positioned an absorbent pad for absorbing exudates from the wound. Finally, a non-stick perforated film layer is normally positioned over the absorbent pad layer, to provide a barrier between the absorbent pad and the wound itself. This allows the wound fluid to move through the perforated layer without sticking to the wound site. Typically the absorbent pad in such bandage does not include any medicinal components, although comparatively recently, bandage manufacturers have started including antibiotic agents on or within bandages to encourage wound healing.

The skin sealant and dye composition of this invention can replace this seemingly complicated bandage construction with a single liquid treatment that will dry to a flexible coating that protects a wound much like a bandage would. Additionally, medicaments such as antibiotic agents may be blended in effective amounts with the composition to provide additional benefits in the area of microbial inhibition and the promotion of wound healing. The sealant composition may be applied to provide an effectively thick coating over the surface of the superficial wound, burn or abrasion. Because the to-be-treated wound is superficial and does not extend beyond the dermal layer, any polymeric residues diffusing into or forming in the wound will be naturally exfoliated from the skin. Generally, the sealant provides a plasticized adhesive film coating over the wound area which when set is satisfactorily flexible and adherent to the tissue without premature peeling or cracking. The coating generally has a thickness of less than about 0.5 millimeter (mm).

Sealant coatings of such thicknesses form a physical barrier layer over superficial wounds which provide protection for the wound in the same manner as a conventional bandage. Specifically, the coating provides an almost airtight, waterproof seal around the wound which does not need to be replaced when the wound gets wet. Once applied, the coating prevents bacterial and contaminant entry into the wound, thus reducing the rate of secondary infection. Generally, the adhesive coating does not limit dexterity and promotes faster wound healing. Additionally, unlike conventional bandages, the sealant naturally sloughs off the skin within 2-3 days after application and, accordingly, avoids the discomfort associated with removal of conventional bandages from the skin. However, if early removal of this polymeric coating is desired, such can be achieved by use of solvents such as acetone. Further discussion of this use may be found in US patent 6,342,213.

By way of elaboration it should be noted that several wound care products are currently being marketed which contain an antiseptic benzalkonium chloride and an antibiotic composition of polymixin B-sulfate and bacitracin-zinc. Patents in this area of technology have described the use of commonly known antiseptics and antibiotics, such as those described in US patents 4,192,299, 4,147,775, 3,419,006, 3,328,259, and 2,510,993. US patent 6,054,523, to Braun et al., describes materials that are formed from organopolysiloxanes containing groups that are capable of condensation, a condensation catalyst, an organopolysiloxane resin, a compound containing a basic nitrogen, and polyvinyl alcohol. US patent 5,112,919, reported a moisture-crosslinkable polymer that was produced by blending a thermoplastic base polymer, such as polyethylene, or a copolymer of ethylene, with 1-butene, 1-hexene, 1-octene, or the like; a solid carrier polymer, such as ethylene vinylacetate copolymer (EVA), containing a silane, such as vinyltrimethoxysilane; and a free-radical generator, such as an organic peroxide; and heating the composition. The copolymers could then be cross-linked by reaction in the presence of water and a catalyst, such as dibutyltin dilaurate, or stannous octoate. US patent 4,593,071 to Keough reported moisture crosslinkable ethylene copolymers having pendant silane acryloxy groups.

A polyurethane wound coating is described by Tedeshchl et al., in EP 0992 252 A2, where a lubricious, drug-accommodating coating is described that is the product of a polyisocyanate; an amine donor, and/or a hydroxyl donor; and an isocyanatosilane adduct having terminal isocyanate groups and an alkoxy silane. A water soluble polymer, such as poly(ethylene oxide), can optionally be present. Cross-linking causes a polyurethane or a polyurea network to form, depending upon whether the isocyanate reacts with the hydroxyl donors or the amine donors. US patent 6,967,261 describes the use of chitosan in wound treatment. Chitosan is a deacetylated product of chitin (C₈ H₁₃ NO₅)ₙ, an abundant natural glucosamine polysaccharide. In particular, chitin is found in the shells of crustaceans, such as crabs, lobsters and shrimp. The compound is also found in the exoskeletons of marine zooplankton, in the wings of certain insects, such as butterflies and ladybugs, and in the cell wall of yeasts, mushrooms and other fungi. Antimicrobial properties of chitosan have been reported against Gram positive and Gram negative bacteria, including Streptococcus spp., Staphylococcus aureus, Staphylococcus epidermidis, Staphylococcus haemolyticus, Pseudomonas, Escherichia, Proteus, Klebsiella, Serratia, Acinobacter, Enterobacter and Citrobacter spp. Chitosan has also been described in the literature to induce repair of tissue containing regularly arranged collagen bundles.

The composition may also be used to close wounds much like stitches or bandages. To be used in such a way, the composition is applied to at least one skin surface of the opposed skin sections of, for example, a suturable wound of a mammalian patient (e.g., human patient). The opposed skin sections are contacted with each either before or after application of the composition. In either case, after application of the composition, the wound area is maintained under conditions wherein the composition polymerizes to join these skin sections together. In general, a sufficient amount of the composition may be employed to cover the wound and the adjacent the skin surface of at least one of the opposed skin sections of the suturable wound. Upon contact with skin moisture and tissue protein, the composition will polymerize or, in the case of compositions utilizing partially polymerized monomers, will further polymerize, at ambient conditions (skin temperature) over about 10 seconds to 60 seconds to provide a solid polymeric film which joins the skin sections, thereby closing the wound. Generally, the composition can provide a plasticized polymeric film over the separated skin sections thereby inhibiting infection of the wound while promoting healing. Further discussion of this use may be found in US patent 6,214,332.

The skin sealant described herein contains one or more phase change dyes which results in a visual color change on resin cure. It is also possible to mix the phase change indicating dye along with a non-color changing dye into the sealant in order to achieve a different series of color changes. These changes in color are due to the mixing of colors to give one color in the liquid phase which changes to totally different color in the cured state due the phase change indicator undergoing a color change resulting in the color composition now giving color 2. By way of illustration, by mixing D&C orange 5 with D&C green 6 into the cyanoacrylate composition gives a green liquid (yellow + green = a rich green). When the resin changes phase and cures the resultant color is purple blue (bright pink + green = purple blue). Thus by carefully selecting the color compositions a variety of color 1 to color 2 changes are possible with this system.

The sealant may be packaged in a "kit" form for use in medical facilities and bundled with the appropriate skin prep solution for ease of use and the convenience of the medical personnel. Kits may also include a container holding the skin sealant composition and another separate container for the dye as previously described. The kit may also include an applicator and means for mixing the contents of the two containers. Alternatively the dye may be impregnated onto a sponge which is used to apply the sealant and through which the skin sealant flows when it is dispensed. In addition, various complimentary or "mating" containers and different packaging schemes have been used for some time and are known in the art.
The following examples show the efficacy of the instant approach. In the Examples below, the cyanoacrylate was mixed with the dye using a stir bar or mechanical stirrer while warmed to and held at 60 °C until the dye dissolved (5-20 minutes).

### Example 1

D&C orange 5 (Sigma-Aldrich Chemical Company, Milwaukee WI) was dissolved into InteguSeal® skin sealant cyanoacrylate resin at a concentration of 200ppm. A drop (30mg) of the composition was transferred to microscope glass slide using a glass rod and then spread over the slide using the glass rod. The initial yellow color liquid changed to deep fluorescent pink on cure. The pink color only formed when the skin sealant was cured and not when it was still wet or tacky. A vividly striking color change signaling cure had been achieved.

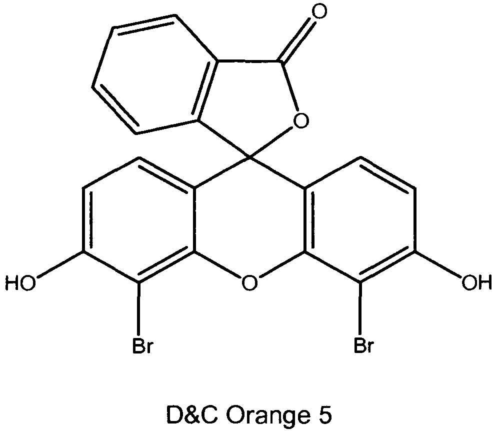

### Example 2

D&C orange 5 was dissolved into InteguSeal® skin sealant cyanoacrylate resin at a concentration of 500ppm. A drop (30mg) of the composition was transferred to microscope giass slide using a glass rod and then spread over the slide using the glass rod. The initial yellow color liquid changed to deep fluorescent pink on cure. The pink color only formed when the skin sealant was cured and not when it was still wet or tacky.

### Example 3

D&C orange 5 was dissolved into InteguSeal® skin sealant cyanoacrylate resin at a concentration of 1000ppm. A drop (30mg) of the composition was transferred to microscope glass slide using a glass rod and then spread over the slide using the glass rod. The initial yellow color liquid changed to deep fluorescent pink on cure. The pink color only formed when the skin sealant was cured and not when it was still wet or tacky.

### Example 4

D&C orange 5 was dissolved into InteguSeal® skin sealant cyanoacrylate resin at a concentration of 5000ppm. A drop (30mg) of the composition was transferred to microscope glass slide using a glass rod and then spread over the slide using the glass rod. The initial yellow color liquid changed to deep fluorescent pink on cure. The pink color only formed when the skin sealant was cured and not when it was still wet or tacky.

### Example 5

D&C orange 5 was dissolved into Hard as Nails tosylamide/formaldehyde based-resin (Del Laboratories Inc., Uniondale NY) at a concentration of 500ppm by simply adding the powder to the resin in a container and stirring the composition for 5mintues to dissolve and ensure the dye was fully homogeneously distributed throughout the resin. A drop (30mg) of the composition was transferred to microscope glass slide using a glass rod and then spread over the slide using the glass rod. The initial yellow color liquid changed to deep fluorescent pink on cure. The pink color only formed when the resin was cured and not when it was still wet or tacky.

### Example 6

D&C orange 5 was dissolved into epoxy resin (Loctite Quick set, Henkel Consumer Adhesives, Inc., Avon OH) at a concentration of 500ppm. A drop (30mg) of the composition was transferred to microscope glass slide using a glass rod and then spread over the slide using the glass rod. The initial yellow color liquid changed to deep fluorescent pink on cure. The pink color only formed when the epoxy resin was cured and not when it was still wet or tacky.

### Example 7

D&C orange 5 was dissolved into Krazy glue (Elmer's Products, Inc., Columbus OH) resin at a concentration of 500ppm. The super glue is an ethyl cyanoacrylate. A drop (30mg) of the composition was transferred to microscope glass slide using a glass rod and then spread over the slide using the glass rod. The initial yellow color liquid changed to deep fluorescent pink on cure. The pink color only formed when the superglue was cured and not when it was still wet or tacky.

### Example 8

D&C orange 5 was dissolved into Scotch super strength adhesive (3M Construction and Home Improvement Market Division, St. Paul MN) at a concentration of 500ppm. A drop (30mg) of the composition was transferred to microscope glass slide using a glass rod and then spread over the slide using the glass rod. The initial yellow color liquid changed to deep fluorescent pink on cure. The pink color only formed when the resin was cured and not when it was still wet or tacky.

### Example 9

D&C orange 5 was dissolved into Liquid Nails styrene/butadiene copolymer-based adhesive (Macco, Cleveland OH) at a concentration of 500ppm. A drop (30mg) of the composition was transferred to microscope glass slide using a glass rod and then spread over the slide using the glass rod. The initial yellow color liquid changed to deep fluorescent pink on cure. The pink color only formed when the rubber cement was cured and not when it was still wet or tacky.

### Example 10

The composition described in example 2 was applied to a mannequin's arm with a small hobby paint brush and spread out to give a thin coating (5cmx4cm). The yellow initial coverage was clearly visible which then turned to a fluorescent pink on cure.

### Example 11

The composition described in example 2 was applied to a mannequin's arm which had been previously coated with Betadine® skin prep (Perdue Pharma, Starnford, Connecticut) using a foam brush applicator. The composition was spread onto the surface with a small hobby paint brush to yield a thin coating. The yellow initial coverage was clearly visible which then turned to a fluorescent pink on cure. The pink color was clearly visible on top of the red-brown skin prep coating.

### Example 12

To the composition described in example 2 was added ascorbic acid (Sigma-Aldrich Chemical Company, Milwaukee WI) to give a 300ppm concentration. A mannequin's arm was coated with Betadine® skin prep using a foam brush applicator. A small sample of the composition was placed on a section of the skin prep treated arm and spread thinly (5cmx6cm) with the brush. The red-brown color of the skin prep was observed to be discharged in under 2minutes (by the ascorbic acid) leaving the yellow color of the liquid cyanoacrylate composition. The yellow color changed to a bright fluorescent pink on cure. Thus the exemplary composition can be mixed with actives that will discharge the color of skin preparations or pretreatments allowing for addition clarity of the application area for the medical staff.

### Example 13

D&C orange 5 was dissolved into InteguSeal® skin sealant to generate 10ml of a yellow solution with a dye concentration of 5000ppm. 5ml of the solution was transferred into an onion-skin glass ampoule and sealed with a rubber plug. This ampoule was loaded into the body of an InteguSeal® applicator, which was then reassembled. The applicator was then activated by pushing the top part of the applicator into the body of the applicator; which resulted in breaking the ampoule inside to release the liquid. The liquid sealant was observed to diffuse through the foam tip and was applied to a mannequin's arm by brushing the foam tip along a section of the arm. The yellow coating was easily observed which turned a deep fluorescent pink color on curing indicating to the user that the sealant coating was dried and ready for further procedures to begin.

### Example 14

To the composition described in example 4 was added green mica-based glitter (from Life of the Party, North Brunswick, NJ) to give a 100ppm concentration and was stirred for 5 minutes to ensure uniform distribution of the mica particles. 40mg of this composition was applied to a microscope glass slide using a glass rod and spread thinly (5cmx5cm). The mica glitter was very visible and clearly indicated coverage area by the deep reflective color. When viewed at a different angle the color vanished allowing good visibility of the skin color and features. The yellow based color of the resin changed to fluorescent pink when the resin had cured. The pink color could easily be seen independently from the green mica glitter.

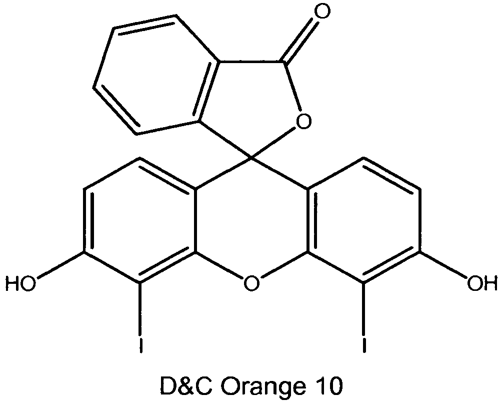

### Example 15

D&C Orange 10 (Sigma-Aldrich Chemical Company, Milwaukee WI) was dissolved into integuSeal® skin sealant cyanoacrylate resin at a concentration of 500ppm. A drop (30mg) of the composition was transferred to a microscope glass slide using a glass stirring rod and then spread across the slide using the glass rod. The initial yellow color changed to an orange pink color on solidifying. The color transformation only occurred on cure of the resin.

### Example 16

D&C Orange 10 (Sigma-Aldrich Chemical Company, Milwaukee WI) was dissolved into Krazy® glue (Elmer's Products, Inc. Columbus OH) resin at a concentration of 500ppm. This glue is an ethyl cyanoacrylate-based resin. A drop (30mg) of the composition was transferred to a microscope glass slide using a glass stirring rod and then spread across the slide using the glass rod. The initial yellow color changed to an orange pink color on solidifying. The color transformation only occurred on cure of the resin.

### Example 17

D&C orange 10 was dissolved into Hard as Nails® tosylamide/formaldehyde based-resin (Del Laboratories Inc., Uniondale NY) at a concentration of 500ppm by simply adding the powder to the resin in a container and stirring the composition for 5mintues to dissolve and ensure the dye was fully homogeneously distributed throughout the resin. A drop (30mg) of the composition was transferred to microscope glass slide using a glass rod and then spread over the slide using the glass rod. The initial yellow color liquid changed to orange-pink color on cure. The pink color only formed when the resin was cured and not when it was still wet or tacky.

### Example 18

D&C orange 5 and 10 are members of the xanthene class of dyes. A study to determine if other members of this class would also exhibit this unique and unexpected property was conducted. In this study, each dye was dissolved into InteguSeal® skin sealant cyanoacrylate resin at a concentration of 500ppm. A drop (30mg) of the composition was transferred to microscope glass slide using a glass rod and then spread over the slide using the glass rod. The resin was observed to determine if a color change on resin cure was observed. If a change in the color was observed then the specific color changes were recorded.

Structures for the dyes are given immediately below and the results of the study are shown in the table below. It can be seen that only the diiodo- and dibromo- members of this class undergo the vivid color change on curing of the cyanoacrylate-based resin.

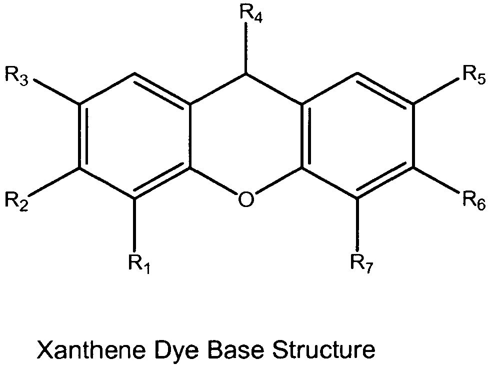

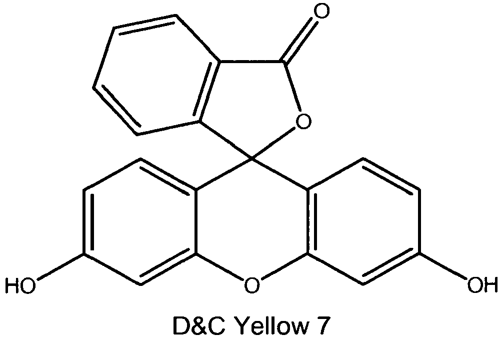

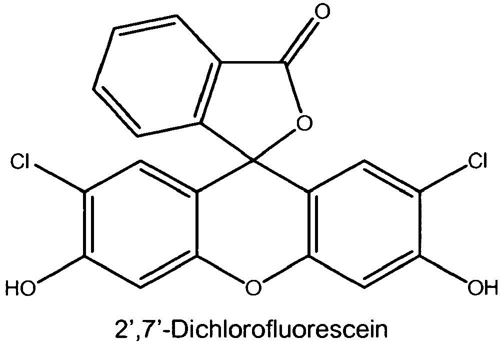

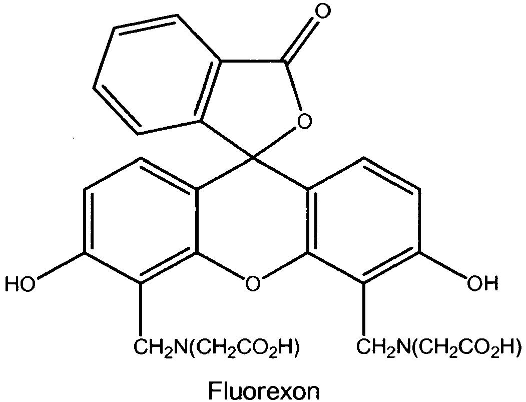

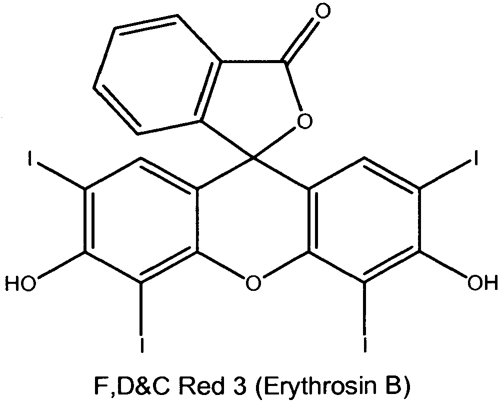

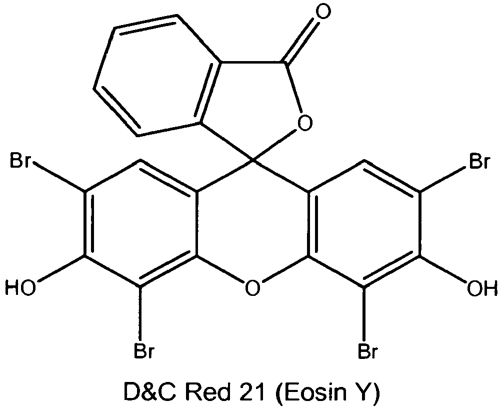

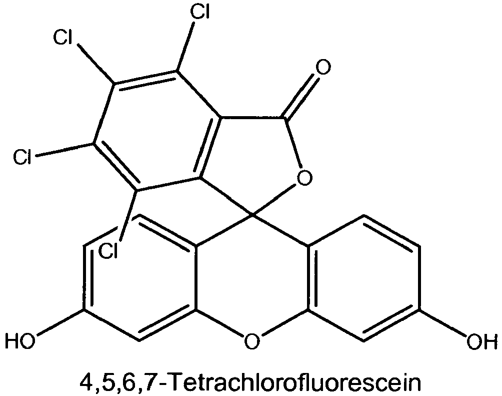

| **Dye** | **Halogen** | **Color Change Observed** |
|---|---|---|
| D&C yellow 7 | None (unsubstituted) | No color change observed on curing |
| D&C orange 5 | 4',5'-dibromo | Yellow to fluorescent pink |
| D&C orange 10 | 4',5'-diiodo | Yellow to orange-pink |
| | 2',7'-dichloro | No color change |
| | | |
| Flurexon | 4',5'-Bis-carboxymethyl | No color change on cure |
| | | |
| D&C red 21 | 2',4',5',7'-Tetrabromo | No color change |
| FD&C red 3 | 2,'4',5',7'-Tetraiodo | No color change |
| | 4,5,6,7-Tetrachloro | No color change |

In addition, a series of other drug & cosmetic (D&C) dyes were tested in the same manner in cyanoacrylate-based sealant to determine if they would undergo a visible color change. As can be seen in the table below none of the other dyes tried changed in color on cure of the resin. This underscores the unexpected property of these unique dyes.

| **Dye** | **Class** | **Color Change on Cure** |
|---|---|---|
| D&C red 22 | xanthene | No color change observed |
| D&C red 30 | benzoth iophenone | No color change observed |
| D&C red 28 | xanthene | No color change observed |
| FD&C yellow 6 | phenylazonaphthol | No color changed observed |
| D&C green 5 | anthraquinone | No color change observed |
| D&C green 6 | anthraquinone | No color change observed |

### Example 19

To the D&C orange 5 composition describe in example 3 was added 10mg of D&C green 6 and the composition stirred to yield a green liquid. A sample (30mg) of this composition was transferred to a microscope slide using a glass rod and the drop spread across the slide. The color of the liquid was visibly observed to change to a purple-blue color on cure of the resin.

### Example 20

To the D&C orange 5 composition described in example 3 was added 10mg of D&C violet 2 and stirred to yield a violet-red liquid. A sample (30mg) of this liquid was transferred using a glass rod to a microscope glass slide and spread thinly on the slide. The color of the liquid was visibly observed to change to a scarlet-purple color of cure of the resin.

As will be appreciated by those skilled in the art, changes and variations to the invention are considered to be within the ability of those skilled in the art. Such changes and variations are intended by the inventors to be within the scope of the invention. It is also to be understood that the scope of the present invention is not to be interpreted as limited to the specific embodiments disclosed herein, but only in accordance with the appended claims when read in light of the foregoing disclosure.

## Claims

1. A system for sealing skin with a film forming polymer, comprising an at least two part applicator (10) containing a sealant composition in one or more frangible containers (4), instructions for using said applicator, the parts being movable such that at least one of said applicator parts (3) moves from a first position to a second position to apply said sealant to skin, wherein said composition comprises a film former wherein said film former comprises cyanoacrylate-based resin, a plasticizer and **characterized in that** said composition further comprises 3000 to 10000 ppm of a dye that changes color when said composition undergoes a phase change, wherein the color change is visible to a human eye under normal light conditions, and wherein said dye comprises dibromo- and diiodo- members of the xanthene class of dyes.

2. The system of claim 1, wherein said first position preserves the integrity of said frangible containers (4).

3. The system of claim 1, wherein said second position does not preserve the integrity of said frangible containers (4).

4. The system of claim 1, wherein said instructions comprise images illustrating said first and second positions.

5. The system of claim 1, wherein said instructions comprise an arrow on an outside surface of said applicator (10) indicating a direction to move said applicator part (3) from said first to said second position.

6. The system of claim 1 for use with skin containing a wound, abrasion, bum, acne, blister and other disruptions.

7. The system of claim 1 further comprising an Iodine-containing skin prep solution.

## Patentansprüche

1. System zum Versiegeln von Haut mit einem filmbildenden Polymer, welches umfasst: einen mindestens zweiteiligen Applikator (10), welcher eine Versiegelungszusammensetzung in einem oder mehreren zerbrechlichen Behältern aufweist; Anweisungen zur Verwendung des Applikators; wobei die Teile beweglich sind, so dass sich mindestens einer der Applikatorteile (3) von einer ersten Position zu einer zweiten Position bewegt, um die Versiegelung auf die Haut aufzutragen, wobei die Zusammensetzung einen Filmbildner umfasst, wobei der Filmbildner ein Cyanoacrylat basiertes Harz und einen Weichmacher umfasst, und **dadurch gekennzeichnet, dass** die Zusammensetzung des Weiteren 3.000 bis 10.000 ppm eines Farbstoffs umfasst, der die Farbe wechselt, wenn die Zusammensetzung einen Phasenwechsel durchmacht, wobei der Farbwechsel unter normalen Lichtverhältnissen für das menschliche Auge sichtbar ist, und wobei der Farbstoff Dibrom- und Diiod-Elemente der Xanthen-Farbstoffklasse umfasst.

2. System gemäß Anspruch 1, wobei die erste Position die Integrität der zerbrechlichen Behälter (4) erhält.

3. System gemäß Anspruch 1, wobei die zweite Position nicht die Integrität der zerbrechlichen Behälter (4) erhält.

4. System gemäß Anspruch 1, wobei die Anweisungen Bilder umfassen, die die erste und zweite Position darstellen.

5. System gemäß Anspruch 1, wobei die Anweisungen einen Pfeil auf einer Außenoberfläche des Applikators (10) umfassen, welcher eine Richtung anzeigt, in welcher der Applikatorteil (3) von der ersten zu der zweiten Position bewegt werden soll.

6. System gemäß Anspruch 1 zur Verwendung mit einer Haut, welche eine Wunde, Abschürfung, Verbrennung, Akne, Blase und andere Störungen aufweist.

7. System gemäß Anspruch 1, welches des Weiteren eine jodhaltige Hautbehandlungslösung umfasst.

## Revendications

1. Système pour protéger la peau avec un polymère filmogène, comprenant un applicateur en au moins deux parties (10) contenant une composition d'agent de protection dans un ou plusieurs récipients cassables (4), des instructions pour utiliser ledit applicateur, les parties étant mobiles de sorte qu'au moins une desdites parties d'applicateur (3) se déplace d'une première position à une seconde position pour appliquer ledit agent de protection sur la peau, dans lequel ladite composition comprend une matière filmogène dans laquelle ladite matière filmogène comprend une résine à base de cyanoacrylate, un plastifiant et **caractérisé en ce que** ladite composition comprend en outre 3 000 à 10 000 ppm d'un colorant qui change de couleur lorsque ladite composition subit un changement de phase, dans lequel le changement de couleur est visible à l'oeil humain sous des conditions lumineuses normales, et dans lequel ledit colorant comprend des éléments dibromo et diiodo de la classe de colorants du xanthène.

2. Système selon la revendication 1, dans lequel ladite première position préserve l'intégrité desdits récipients cassables (4).

3. Système selon la revendication 1, dans lequel ladite seconde position ne préserve pas l'intégrité desdits récipients cassables (4).

4. Système selon la revendication 1, dans lequel lesdites instructions comprennent des images illustrant lesdites première et seconde positions.

5. Système selon la revendication 1, dans lequel lesdites instructions comprennent une flèche sur une surface extérieure dudit applicateur (10) indiquant une direction pour déplacer ladite partie d'applicateur (3) de ladite première à ladite seconde position.

6. Système selon la revendication 1 pour une utilisation avec une peau contenant une plaie, une abrasion, une brûlure, de l'acné, une cloque et d'autres interruptions.

7. Système selon la revendication 1, comprenant en outre une solution de préparation cutanée contenant de l'iode.
